Europäisches Patentamt
European Patent Office
Office européen des brevets

(19) (11) Publication number: **0 135 814 A1**

(12) **EUROPEAN PATENT APPLICATION**

(21) Application number: 84110082.9

(22) Date of filing: 23.08.84

(51) Int. Cl.⁴: C 07 D 403/12, C 07 D 417/14 // C07D205/08

(30) Priority: 26.08.83 US 526837

(43) Date of publication of application: 03.04.85 Bulletin 85/14

(84) Designated Contracting States: AT BE CH DE FR GB IT LI LU NL SE

(71) Applicant: E.R. Squibb & Sons, Inc.,
Lawrenceville-Princeton Road, Princeton,
N.J. 08540 (US)

(72) Inventor: Hoehn, Hans, Arberstrasse 12,
D-8401 Tegernheim (DE)
Inventor: Breuer, Hermann, Sauerzapfstrasse 5,
D-8411 Schoenhofen (DE)

(74) Representative: Vossius Vossius Tauchner Heunemann
Rauh, Siebertstrasse 4 P.O. Box 86 07 67,
D-8000 München 86 (DE)

(54) **1-(1H-tetrazol-5-ylalkoxy)-2-azetidinones.**

(57) Antibacterial activity is exhibited by 3-acylamino-2-azetidinones having in the 1-position a group of the formula

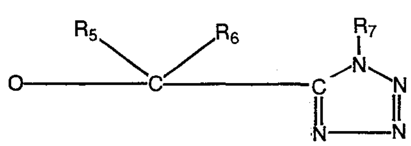

...IUS · VOSSIUS · TAUCHNER
HEUNEMANN · RAUH
PATENTANWÄLTE
...ERTSTR. 4, 8000 MÜNCHEN 80
TEL. (089) 47 40 75

Our Ref: T 194 EP
Case: V-526,837-H
E.R. Squibb & Sons, Inc.
Princeton, N.J. 08540, USA

August 23, 1984

# 1-(1H-TETRAZOL-5-YLALKOXY)-2-AZETIDINONES

Compounds having the formula

I

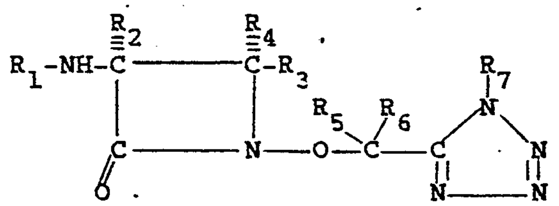

and salts thereof, exhibit antibacterial activity. In formula I, and throughout the specification, the symbols are as defined below.

$R_1$ is an acyl group derived from a carboxylic acid;

$R_2$ is hydrogen or methoxy;

$R_3$ and $R_4$ are the same or different and each is hydrogen, alkyl, alkenyl, alkynyl, cycloalkyl, phenyl, substituted phenyl or a 4, 5, 6 or 7-membered heterocycle (referred to hereinafter as $R_x$) or one of $R_3$ and $R_4$ is hydrogen and the other is azido, halomethyl, dihalomethyl, trihalomethyl, alkoxycarbonyl, 2-phenylethenyl, 2-phenylethynyl, carboxyl, $-CH_2X_1$ [wherein $X_1$ is azido, amino ($-NH_2$), hydroxy, alkanoylamino, phenylcarbonylamino, (substituted phenyl)carbonylamino, alkylsulfonyloxy, phenylsulfonyloxy, (substituted phenyl)sulfonyloxy, phenyl, substituted phenyl, cyano, $-A-\overset{O}{\overset{\|}{C}}-NX_6X_7$, $-S-X_2$, or $-O-X_2$ (wherein A, $X_2$, $X_6$ and $X_7$ are as hereinafter defined)], $-S-X_2$ or $-O-X_2$ [wherein $X_2$ is alkyl, substituted alkyl, phenyl, substituted phenyl, phenylalkyl, (substituted phenyl)alkyl, alkanoyl, phenylalkanoyl, (substituted phenyl)alkanoyl, phenylcarbonyl,

(substituted phenyl)carbonyl, or heteroaryl-carbonyl], 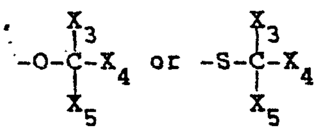 [wherein one of $X_3$ and $X_4$ is hydrogen and the other is hydrogen or alkyl, or $X_3$ and $X_4$ when taken together with the carbon atom to which they are attached form a cycloalkyl group; and $X_5$ is formyl, alkanoyl, phenylcarbonyl, (substituted phenyl)carbonyl, phenylalkylcarbonyl, (substituted phenyl)alkylcarbonyl, carboxyl, alkoxycarbonyl, aminocarbonyl (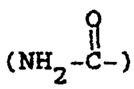), (substituted amino)-carbonyl, or cyano ($-C\equiv N$)], or 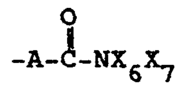 (wherein A is $-CH=CH-$, $-(CH_2)_n-$, $-CH_2-O-$, $-CH_2-NH-$, or $-CH_2-S-CH_2-$, n is 0, 1 or 2, and $X_6$ and $X_7$ are the same or different and each is hydrogen, alkyl, phenyl or substituted phenyl, or $X_6$ is hydrogen and $X_7$ is amino, substituted amino, acylamino or alkoxy, or $X_6$ and $X_7$ when taken together with the nitrogen atom to which they are attached form a 4, 5, 6 or 7-membered heterocycle);

$R_5$ and $R_6$ are the same or different and each is hydrogen, alkyl, alkenyl, alkynyl, phenyl, substituted phenyl, cycloalkyl or $R_x$, or $R_5$ and $R_6$ together with the carbon atom to which they are attached are cycloalkyl or $R_x$, or one of $R_5$ and $R_6$ is hydrogen and the other is azido, halomethyl, dihalomethyl, trihalomethyl, halogen, alkoxycarbonyl, alkenyl, alkynyl, 2-phenylethenyl,

2-phenylethynyl, carboxyl, $-CH_2-X_1$, $-S-X_2$, $-O-X_2$, or $-A-\overset{O}{\overset{\|}{C}}-NX_6X_7$; and

$R_7$ is hydrogen, alkyl, phenyl, substituted phenyl, phenylalkyl or (substituted phenyl)alkyl.

Listed below are definitions of various terms used to describe the β-lactams of this invention. These definitions apply to the terms as they are used throughout the specification (unless they are otherwise limited in specific instances) either individually or as part of a larger group.

The terms "alkyl" and "alkoxy" refer to both straight and branched chain groups. Those groups having 1 to 10 carbon atoms are preferred.

The terms "cycloalkyl" and "cycloalkenyl" refer to cycloalkyl and cycloalkenyl groups having 3,4,5,6 or 7 carbon atoms.

The term "substituted alkyl" refers to alkyl groups substituted with one, or more, azido, amino ($-NH_2$), halogen, hydroxy, carboxy, cyano, alkoxycarbonyl, aminocarbonyl, alkanoyloxy, alkoxy, phenyloxy, (substituted phenyl)oxy, $R_x$-oxy, mercapto, alkylthio, phenylthio, (substituted phenyl)thio, alkylsulfinyl, or alkylsulfonyl gruops.

The terms "alkanoyl", "alkenyl", and "alkynyl" refer to both straight and branched chain groups. Those groups having 2 to 10 carbon atoms are preferred.

The terms "halogen" and "halo" refer to fluorine, chlorine, bromine and iodine.

The term "protected carboxyl" refers to a carboxyl group which has been esterified with a conventional acid protecting group. These groups

are well known in the art; see, for example, United States patent 4,144,333, issued March 13, 1979. The preferred protected carboxyl groups are benzyl, benzhydryl, t-butyl, and p-nitrobenzyl esters.

The term "substituted phenyl" refers to a phenyl group substituted with 1, 2 or 3 amino ($-NH_2$), halogen, hydroxyl, trifluoromethyl, alkyl (of 1 to 4 carbon atoms), alkoxy (of 1 to 4 carbon atoms), or carboxyl groups.

The expression "a 4,5,6 or 7-membered heterocycle" (referred to as "$R_x$") refers to substituted and unsubstituted, aromatic and non-aromatic groups containing one or more nitrogen, oxygen or sulfur atoms. Exemplary substituents are oxo (=O), halogen, hydroxy, nitro, amino, cyano, trifluoromethyl, alkyl of 1 to 4 carbons, alkoxy of 1 to 4 carbons, alkylsulfonyl, phenyl, substituted phenyl, 2-furfurylideneamino ( 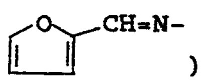 ), benzylideneimino and substituted alkyl groups (wherein the alkyl group has 1 to 4 carbons). One type of "4,5,6 or 7-membered heterocycle" is the "heteroaryl" group. The term "heteroaryl" refers to those 4,5,6 or 7-membered heterocycles which are aromatic. Exemplary heteroaryl groups are substituted and unsubstituted pyridinyl, furanyl, pyrrolyl, thienyl, 1,2,3,-triazolyl, 1,2,4-triazolyl, imidazolyl, thiazolyl, thiadiazolyl, pyrimidinyl, oxazolyl, triazinyl, and tetrazolyl. Exemplary nonaromatic

heterocycles (i.e., fully or partially saturated heterocyclic groups) are substituted and unsubstituted azetinyl, oxetanyl, thietanyl, piperidinyl, piperazinyl, imidazolidinyl, oxazolidinyl, pyrrolidinyl, tetrahydropyrimidinyl, dihyrothiazolyl and hexahydroazepinyl. Exemplary of the substituted 4,5,6 or 7-membered heterocycles are 1-alkyl-3-azetinyl, 2-oxo-1-imidazolidinyl, 3-alkylsulfonyl-2-oxo-1-imidazolidinyl, 3-benzylimino-2-oxo-1-imidazolidinyl, 3-alkyl-2-oxo-1-imidazolidinyl, 3-phenyl (or substituted phenyl)-2-oxo-1-imidazolidinyl, 3-benzyl-2-oxo-1-imidazolidinyl, 3-(2-aminoethyl)-2-oxo-1-imidazolidinyl, 3-amino-2-oxo-1-imidazolidinyl, 3-[(alkoxycarbonyl)amino]-2-oxo-1-imidazolidinyl, 3-[2-[(alkoxycarbonyl)-amino]ethyl]-2-oxo-1-imidazolidinyl, 2-oxo-1-pyrrolidinyl, 2-oxo-3-oxazolidinyl, 4-hydroxy-6-methyl-2-pyrimidinyl, 2-oxo-1- hexahydroazepinyl, 2-oxo-3-pyrrolidinyl, 2-oxo-3-tetrahydrofuranyl, 2,3-dioxo- 1-piperazinyl, 2,5-dioxo-1-piperazinyl, 4-alkyl- 2,3-dioxo-1-piperazinyl, and 4-phenyl-2,3-dioxo-1-piperazinyl.

The term "substituted amino" refers to a group having the formula $-NY_1Y_2$ wherein $Y_1$ is hydrogen, alkyl, phenyl, substituted phenyl, phenylalkyl or (substituted phenyl)alkyl, and $Y_2$ is alkyl, phenyl, substituted phenyl, phenylalkyl, (substituted phenyl)alkyl, hydroxy, cyano, alkoxy, phenylalkoxy, or amino ($-NH_2$).

The term "acyl" refers to all organic radicals derived from an organic acid (i.e., a carboxylic acid) by removal of the hydroxyl group. Certain acyl groups are, of course, preferred but this preference should not be viewed as a limitation of the scope of this invention. Exemplary acyl groups are those acyl groups which have been used in the past to acylate β-lactam antibiotics including 6-aminopenicillanic acid and derivatives and 7-aminocephalosporanic acid and derivatives; see, for example, Cephalosporins and Penicillins, edited by Flynn, Academic Press (1972), German Offenlegungsschrift 2,716,677, published October 10, 1978, Belgian patent 867,994, published December 11, 1978, United States patent 4,152,432, issued May 1, 1979, United States patent 3,971,778, issued July 27, 1976, United States patent 4,172,199, issued October 23, 1979, and British patent 1,348,894, published March 27, 1974. The following list of acyl groups is presented to further exemplify the term "acyl"; it should not be regarded as limiting that term.

Exemplary acyl groups are:

(a) Aliphatic groups having the formula

$$R_a-\overset{\overset{\displaystyle O}{\|}}{C}-$$

wherein $R_a$ is alkyl; cycloalkyl; alkoxy; alkenyl;

cycloalkenyl; cyclohexadienyl; or alkyl or alkenyl substituted with one or more halogen, cyano, nitro, amino, mercapto, alkylthio, or cyanomethylthio groups.

(b) Carbocyclic aromatic groups having the formula

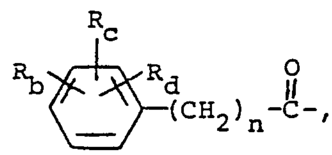

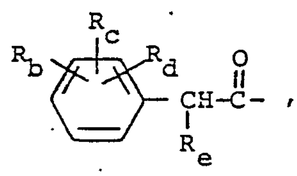

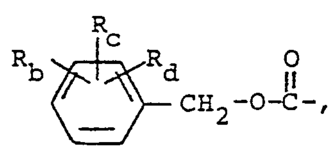

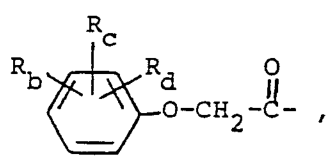

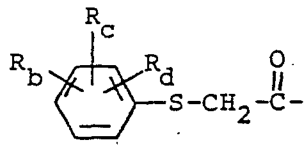 or

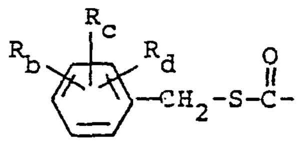

wherein n is 0, 1, 2 or 3; $R_b$, $R_c$, and $R_d$ each is independently hydrogen, halogen, hydroxyl, nitro, amino, cyano, trifluoromethyl, alkyl of 1 to 4 carbon atoms, alkoxy of 1 to 4 carbon atoms or aminomethyl; and $R_e$ is amino, hydroxyl, a carboxyl salt, protected carboxyl, formyloxy, a sulfo salt, a sulfoamino salt, azido, halogen, hydrazino, alkylhydrazino, phenylhydrazino, or [(alkylthio)thioxomethyl]thio.

Preferred carbocyclic aromatic acyl groups include those having the formula

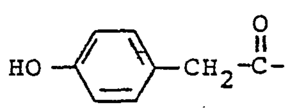,

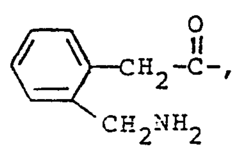,

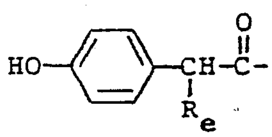 ($R_e$ is preferably a carboxyl salt or sulfo salt) and

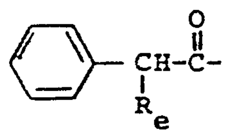 ($R_e$ is preferably a carboxyl salt or sulfo salt).

(c) Heteroaromatic groups having the formula

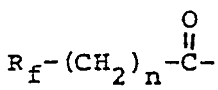,

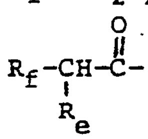,

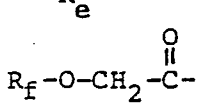,

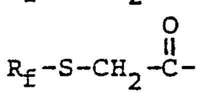,

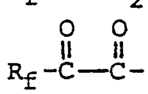,

wherein n is 0, 1, 2 or 3; $R_e$ is as defined above; and $R_f$ is a substituted or unsubstituted 5-, 6- or 7-membered heterocyclic ring containing 1,2,3 or 4 (preferably 1 or 2) nitrogen, oxygen and sulfur atoms. Exemplary heterocyclic

rings are thienyl, furyl, pyrrolyl, pyridinyl, pyrazolyl, pyrazinyl, thiazolyl, pyrimidinyl, thiadiazolyl and tetrazolyl. Exemplary substituents are halogen, hydroxyl, nitro, amino, protected amino, cyano, trifluoromethyl, alkyl of 1 to 4 carbon atoms, alkoxy of 1 to 4 carbon atoms, or

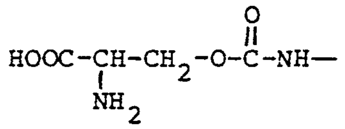 .

Preferred heteroaromatic acyl groups include those groups of the above formulas wherein $R_f$ is 2-amino-4-thiazolyl, 2-amino-5-halo-4-thiazolyl, 4-aminopyrimidin-2-yl, 5-amino-1,2,4-thiadiazol-3-yl, 2-thienyl, 2-furanyl, or 6-aminopyridin-2-yl.

(d) [[(4-Substituted-2,3-dioxo-1-piperazinyl)carbonyl]amino]arylacetyl groups having the formula

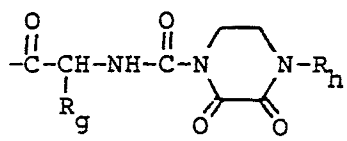

wherein $R_g$ is an aromatic group (including carbocyclic aromatics such as those of the formula

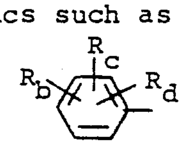

and heteroaromatics as included within the definition of $R_f$); and $R_h$ is alkyl, substituted

alkyl (wherein the alkyl group is substituted with one or more halogen, cyano, nitro, amino or mercapto groups), arylmethyleneamino (i.e., $-N=CH-R_g$ wherein $R_g$ is as defined above), arylcarbonylamino (i.e., $-NH-\overset{O}{\overset{\|}{C}}-R_g$ wherein $R_g$ is as defined above) or alkylcarbonylamino.

Preferred [[(4-substituted-2,3-dioxo-1-piperazinyl)carbonyl]amino]arylacetyl groups include those wherein $R_h$ is ethyl, phenylmethyleneamino or 2-furylmethyleneamino.

(e) (Substituted oxyimino)arylacetyl groups having the formula

$$-\overset{O}{\overset{\|}{C}}-\underset{R_g}{\underset{|}{C}}=N-O-R_i$$

wherein $R_g$ is as defined above and $R_i$ is hydrogen, alkyl, cycloalkyl, alkylaminocarbonyl, arylaminocarbonyl (i.e., $-\overset{O}{\overset{\|}{C}}-NH-R_g$ wherein $R_g$ is as defined above) or substituted alkyl (wherein the alkyl group is substituted with one or more halogen, cyano, nitro, amino, mercapto, alkylthio, aromatic group (as defined by $R_g$), carboxyl (including salts thereof), amido, alkoxycarbonyl, phenylmethoxycarbonyl, diphenylmethoxycarbonyl, hydroxyalkoxyphosphinyl, dihydroxyphosphinyl, hydroxy(phenylmethoxy)phosphinyl, or dialkoxyphosphinyl substituents).

Preferred (substituted oxyimino)arylacetyl groups include those wherein $R_g$ is 2-amino-4-thiazolyl. Also preferred are those groups wherein $R_i$ is methyl, ethyl, carboxymethyl, 1-carboxy-1-methylethyl, 2,2,2-trifluoroethyl or 1-carboxycyclopropyl.

(f) (Acylamino)arylacetyl groups having the formula

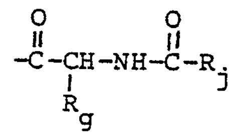

wherein $R_g$ is as defined above and $R_j$ is

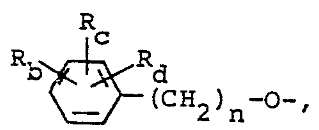, amino, alkylamino, (cyanoalkyl)-amino, amido, alkylamido, (cyanoalkyl)amido,

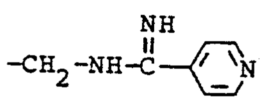

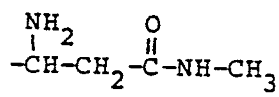,

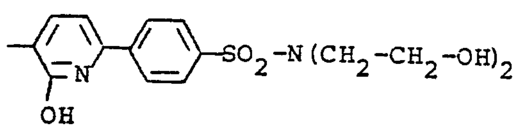,

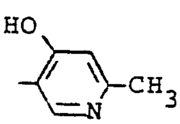,

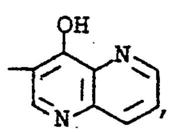,

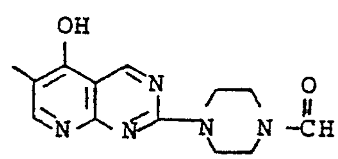, or

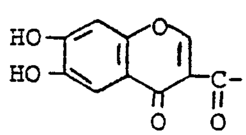.

Preferred (acylamino)arylacetyl groups of the above formula include those groups wherein $R_j$ is amino or amido. Also preferred are those groups wherein $R_g$ is phenyl or 2-thienyl.

(g) [[[3-Substituted-2-oxo-1-imidazolidinyl]carbonyl]amino]arylacetyl groups having the formula

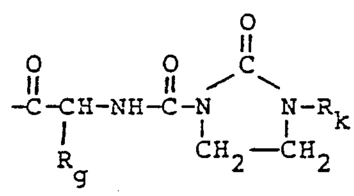

wherein $R_g$ is as defined above and $R_k$ is hydrogen, alkylsulfonyl, arylmethyleneamino (<u>i.e.</u>, $-N=CH-R_g$ wherein $R_g$ is as defined above), $-\overset{O}{\overset{\|}{C}}-R_m$ (wherein $R_m$ is hydrogen, alkyl or halogen substituted alkyl), aromatic group (as defined by $R_g$ above), alkyl or substituted alkyl (wherein the alkyl group is substituted with one or more halogen, cyano, nitro, amino or mercapto groups).

Preferred [[3-substituted-2-oxo-1-imidazolidinyl]carbonyl]amino]arylacetyl groups of the above formula include those wherein $R_g$ is phenyl or 2-thienyl. Also preferred are those groups wherein $R_k$ is hydrogen, methylsulfonyl, phenylmethyleneamino or 2-furylmethyleneamino.

The terms "salt" and "salts" refer to basic salts formed with inorganic and organic bases. Such salts include ammonium salts, alkali metal salts like sodium and potassium salts (which are preferred), alkaline earth metal salts like the calcium and magnesium salts, salts with organic bases, e.g., dicyclohexylamine salt, benzathine, N-methyl-D-glucamine, hydrabamine salts, salts with amino acids like arginine, lysine and the like. The nontoxic, pharmaceutically acceptable salts are preferred, although other salts are also useful, e.g., in isolating or purifying the product.

The salts are formed in conventional manner by reacting the free acid form of the product with one or more equivalents of the appropriate base providing the desired cation in a solvent or medium in which the salt is insoluble, or in water and removing the water by freeze drying. By neutralizing the salt with an insoluble acid like a cation exchange resin in the hydrogen form (e.g., polystyrene sulfonic acid resin like Dowex 50) or with an aqueous acid and extraction with an organic solvent, e.g., ethyl acetate, dichloromethane or the like, the free acid form can be obtained, and, if desired, another salt formed.

β-Lactams of formula I contain at least one chiral center -- the carbon atom (in the 3-position of the β-lactam nucleus) to which the acylamino substituent is attached. This invention is directed to those β-lactams which have been described above, wherein the stereochemistry at the chiral center in the 3-position of the β-lactam nucleus is the same as the configuration at the carbon atom in the 6-position of naturally

occurring penicillins (e.g., penicillin G) and as the configuration at the carbon atom in the 7-position of naturally occuring cephamycins (e.g., cephamycin C).

Also included within the scope of this invention are racemic mixtures which contain the above-described ß-lactams.

The ß-lactams of formula I, and salts thereof, have activity against a range of gram-negative and gram-positive organisms. The compounds of this invention can be used as agents to combat bacterial infections (including urinary tract infections and respiratory infections) in mammalian species, such as domesticated animals (e.g., dogs, cats, cows, horses, and the like) and humans.

For combating bacterial infections in mammals a compound of this invention can be administered to a mammal in need thereof in an amount of about 1.4 mg/kg/day to about 350 mg/kg/day, preferably about 14 mg/kg/day to about 100 mg/kg/day. All modes of administration which have been used in the past to deliver penicillins and cephalosporins to the site of the infection are also contemplated for use with the novel family of ß-lactams of this invention. Such methods of administration include oral, intravenous, intramuscular, and as a suppository.

The ß-lactams of this invention can be prepared from an amino acid having the formula

II

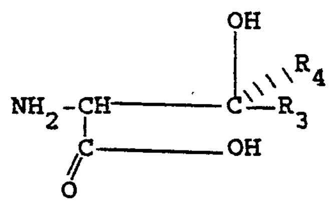

The amino group is first protected with a classical protecting group (e.g., t-butoxycarbonyl, benzyloxycarbonyl, o-nitrophenylsulfenyl, etc.), yielding the compound having the formula

III

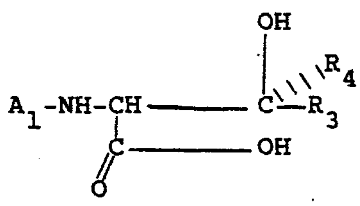

In formula III, and throughout the specification, the symbol "$A_1$" refers to a nitrogen protecting group. For certain products of formula I, the desired acyl group "$R_1$" can be used as the protecting group "$A_1$" and thus incorporated at the beginning of the reaction sequence.

The carboxyl group of a protected amino acid of formula III is then reacted with an amine having the formula

IV

$$Y\text{-}O\text{-}NH_2 \quad .$$

In formula IV, and throughout the specification, the symbol "Y" refers to benzyl, pivaloyl, $-CH_2CH(NHA_2)CO_2$alkyl, t-butyl, p-nitrobenzyl, benzyhydryl, 2-cyanoethyl, 2-trimethylsilylethyl, trichloroethyl, p-anisyl, inter alia (wherein the symbol "$A_2$" refers to a nitrogen protecting group). The reaction proceeds in the presence of a coupling agent such as 1-ethyl-3-(3-dimethylaminopropyl)carbodiimide or dicyclohexylcarbodiimide,

and yields a compound having the formula

V

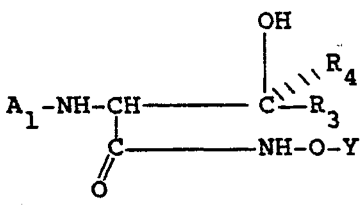

.

The hydroxyl group of a compound of formula V is converted to a leaving group, using, for example, a classical reagent such as methanesulfonyl chloride (methanesulfonyl is referred to hereinafter as "Ms").

The fully protected compound having the formula

VI

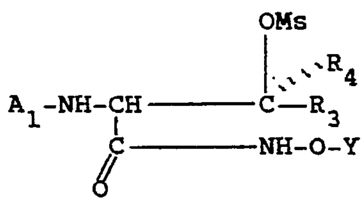

is cyclized by treatment with base, e.g., potassium carbonate. The reaction is preferably carried out in an organic solvent such as acetone, under reflux conditions, and yields a compound having the formula

VII

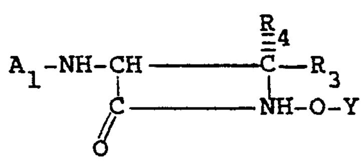

Compounds of formula VII wherein Y is benzyl are disclosed in the literature; see, for example, J.A.C.S., 104:6054 (1982).

Alternatively, cyclization of a compound of formula V can be accomplished without first converting the hydroxyl group to a leaving group. Treatment of a compound of formula V with triphenylphosphine and diethylazodicarboxylate or

carbon tetrachloride, yields a compound of formula VII.

Both of the methods disclosed above for ring closure of a compound of formula V result in the inversion of the stereochemistry of the $R_3$ and $R_4$ substituents.

Selective reduction of a compound of formula VII (using catalytic hydrogenation if Y is benzyl or by treatment with a base such as sodium sulfide or sodium hydroxide if Y is pivaloyl or with DBU if Y is $-CH_2CH(NHA_2)CO_2$alkyl yields the corresponding compound having the formula

VIII

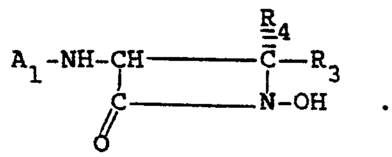

Alkylation of a hydroxamic acid of formula VIII with a compound having the formula

IX

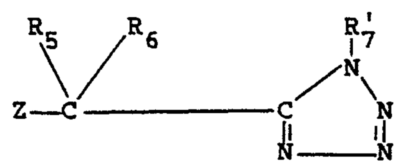

wherein Z is a suitable leaving group, such as a halogen atom (preferably bromine or chlorine), a mesylate or triflate group, or any one of the other leaving groups well known in the art, and $R_7'$ is alkyl, phenyl, substituted phenyl, phenylalkyl, or (substituted phenyl)alkyl, yields the corresponding compound having the formula

X

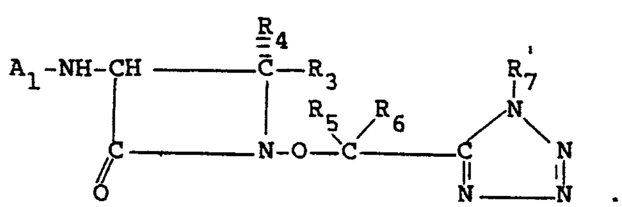

To obtain a compound having the formula

XI

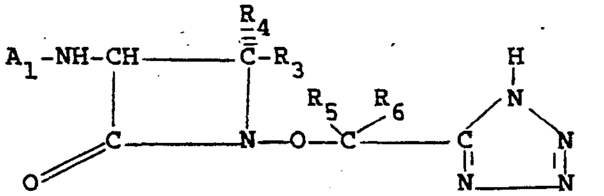

the corresponding compound of formula X, wherein $R_7'$ is benzyl can be treated with hydrogen in the presence of a catalyst (e.g., palladium on charcoal).

Tetrazole derivatives of formula IX are disclosed in the literature; see, for exaple, J. Org. Chem., 17:1597 (1952).

Deprotection of the 3-amino substitutent of a compound of formula X or XI can be accomplished using art-recognized techniques. If, for example, the protecting group is t-butoxycarbonyl, trifluoro- acetic acid can be used to deprotect the amino group. If the protecting group is benzyloxycarbonyl, catalytic (e.g., palladium on charcoal) hydrogenation can be used. If the protecting group is o-nitrophenylsulfenyl, p-toluenesulfonic acid can be used in combination with p-thiocresol. The deprotected compound has the formula

XII

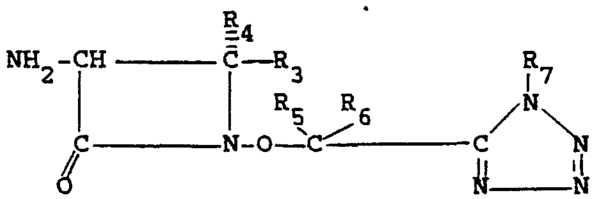

and is a key intermediate for preparing the compounds of this invention. The compounds of formula XII form an integral part of this invention.

Well known acylation techniques can be used to convert a compound of formula XII to the corresponding compound having the formula

XIII

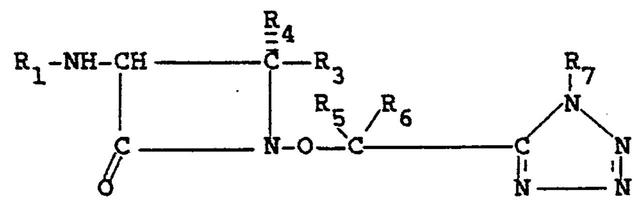

Exemplary techniques include reaction with a carboxylic acid ($R_1$-OH) or corresponding carboxylic acid halide or carboxylic acid anhydride. The reactions with a carboxylic acid proceed most readily in the presence of a carbodiimide such as dicyclohexylcarbodiimide and a substance capable of forming a reactive intermediate <u>in situ</u> such as N-hydroxybenzotriazole or N-hydroxysuccinimide. Alternatively, the reaction with a carboxylic acid can be run in the presence of N-methyl-N-(trimethylsilyl)trifluoroacetamide (MSTFA) and a substance capable of forming a reactive intermediate <u>in situ</u> such as N-hydroxybenzotriazole. In those instances wherein the acyl group ($R_1$) contains reactive functionality (such as amino or carboxyl groups) it may be necessary to first protect these functional groups, then carry out the acylation reaction, and finally deprotect the resulting product.

The products of formula I wherein $R_2$ is methoxy can be prepared from the corresponding compound of formula VII. Halogenating (preferably chlorinating) the amide nitrogen of a compound of formula VII yields a compound having the formula

XIV 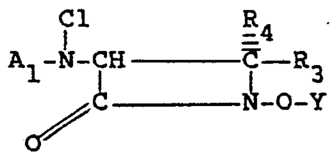 .

Reagents and procedures of N-chlorinating amides are well known in the art. Exemplary reagents are <u>tert</u>.-butyl hypochlorite, sodium hypochlorite, and chlorine. The reaction can be run in an organic solvent (<u>e.g.</u>, a lower alkanol such as methanol) or in a two phase solvent system (<u>e.g.</u>, water/methylene chloride) in the presence of a base such as sodium borate decahydrate. The reaction is preferably run at a reduced temperature.

Reaction of a compound of formula XIV with a methoxylating agent, <u>e.g.</u>, an alkali metal methoxide, yields a compound (in combination with its enantiomer if $R_3$ and $R_4$ are the same or if XIV is a racemic mixture) having the formula

XV 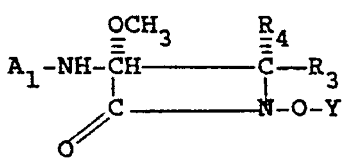 .

The reaction can be run in an organic solvent, <u>e.g.</u>, a polar organic solvent such as tetrahydrofuran, at a reduced temperature.

Alternatively, a compound of formula VII can be converted to a compound of formula XV using a single step procedure. The methoxylating agent can first be mixed with a compound of formula VII and the N-chlorinating reagent then added to the reaction mixture.

Conversion of a compound of formula XV to the desired product of formula I can be accomplished using the procedures described above for the conversion of an intermediate of formula VII to a product of this invention.

The following examples are specific embodiments of this invention.

Example 1

(3S-trans)-3-[(t-Butyloxycarbonyl)amino]-4-methyl-2-oxo-1-[1-(phenylmethyl)-1H-tetrazol-5-ylmethoxy]-azetidine

A) (3S-trans)-3-(t-Butyloxycarbonylamino)-1-hydroxy-4-methyl-2-azetidinone

10.5g of (3S-*trans*)-3-(*t*-Butoxycarbonyl-amino)-1-benzyloxy-4-methyl-2-azetidinone (34.2 mmol) was dissolved in 250ml of methanol and hydrogenated with 1.43g of palladium on carbon (10%) as the catalyst. After 45 minutes, the catalyst was filtered off and the filtrate was evaporated *in vacuo*. The crystalline title compound was dried *in vacuo* at room temperature yielding 7.3g of the title compound, melting point 161-162°C, *dec*.

B) (3S-trans)-3-[(t-Butyloxycarbonyl)amino]-4-methyl-2-oxo-1-[1-(phenylmethyl)-1H-tetrazol-5-ylmethoxy]azetidinone

1.83g of Triethylamine (18.0 mmol) was dropped into a solution of 3.0g of (3S-*trans*)-3-(*t*-butyloxycarbonylamino)-1-hydroxy-4-methyl 2-azetidinone (14.0 mmol) and 2.9g of 5-chloromethyl-1-phenylmethyltetrazole (14.0 mmol) in 90ml of dry dimethylformamide. The mixture was stirred at room temperature for 3 days. The precipitate was filtered off and the solvent was stripped *in vacuo*. The residual oil was taken up in ethyl acetate/ether (1:1) filtered again and chromatographed on silica gel with ethyl acetate/ether (1:1) yielding 5.16g of oily product that crystallized after standing overnight.

## Example 2

(3S-trans)-3-[(t-Butyloxycarbonyl)amino]-4-methyl-2-oxo-1-[1H-tetrazol-5-ylmethoxy]azetidine

2.3g of (3S-*trans*)-3-[(*t*-Butyloxycarbonyl)-amino-4-methyl-2-oxo-1-[1-(phenylmethyl)-1*H*-tetrazol-5-ylmethoxy]azetidine (6.0 mmol) was dissolved in 100ml of dry tetrahydrofuran and hydrogenated with 1.0g of palladium on carbon (10%) as the catalyst. After 20 hours, the catalyst was filtered off and the filtrate was stripped *in vacuo*. Chromatography on silica gel with ethyl acetate/ether (1:1) gave 1.5g of the desired product, melting point 88-90°C.

## Example 3

(3S-trans)-3-[(t-Butyloxycarbonyl)amino]-4-methyl-2-oxo-1-[1H-tetrazol-5-yl-(1-ethoxy)]-azetidine

A) (3S-trans)-3-[(t-Butyloxycarbonyl)amino]-4-methyl-2-oxo-1-[1-(phenylmethyl)-1H-tetrazol-5-yl(1-ethoxy)]azetidine

Following the procedure of example 1B, but substituting 5-(1-chloroethyl)-1-phenylmethyltetrazole for 5-chloromethyl-1-phenylmethyl tetrazole yielded after chromatography (ethyl acetate/ether, 1:1) the title compound as an oil.

B) (3S-trans)-3-[(t-Butyloxycarbonyl)amino]-4-methyl-2-oxo-1-[1H-tetrazol-5-yl(1-ethoxy)]azetidine

Hydrogenation of 3g of (3S-*trans*)-3-[(*t*-butyloxycarbonyl)amino]-4-methyl-2-oxo-1-[1-(phenylmethyl)-1*H*-tetrazol-5-yl(1-ethoxy)]azetidine, dissolved in tetrahydrofuran, with 1.0g of

palladium on carbon (10%) for 5 hours, and work up according to the procedure of example 2, yielded 2.1g of the title compound as an oil.

### Example 4

[3S-[3α(Z),4β]]-2-Amino-α-(methoxyimino)-N-[4-methyl-2-oxo-1-[[1-(phenylmethyl)-1H-tetrazol-5-yl-methoxy]-3-azetidinyl]-4-thiazoleacetamide

A) (3S-trans)-3-Amino-4-methyl-2-oxo-1-[1-(phenylmethyl)-1H-tetrazol-5-ylmethoxy]azetidine, trifluoroacetate

2.0g of (3S-trans)-3-[(t-Butyloxycarbonyl)-amino]-4-methyl-2-oxo-1-[1-(phenylmethyl)-1H-tetrazol-5-ylmethoxy]azetidine (example 1) was dissolved in a mixture of 20ml of trifluoroacetic acid and 2ml of anisole at -10°C. Stirring was continued at 0°C to -5°C for 25 minutes. The solution was evaporated in vacuo at 0°C, the oily residue treated three times with anhydrous ether, and the solid β-lactam was filtered off and dried over $P_2O_5$ to give 1.6g of the title compound.

B) [3S-[3α(Z),4β]]-2-Amino-α-(methoxyimino)-N-[4-methyl-2-oxo-1-[[1-(phenylmethyl)-1H-tetrazol-5-ylmethoxy]-3-azetidinyl]-4-thiazoleacetamide

1.4ml of MSTFA (7.5 mmol) was added to a solution of 1.0g of (3S-trans)-3-amino-4-methyl-2-oxo-1-[1-(phenylmethyl)-1H-tetrazol-5-ylmethoxy]-azetidine, trifluoroacetate (2.5 mmol) in 20ml of dry acetonitrile at 0°C. Stirring was continued for 4.5 hours at room temperature. After evaporation in vacuo, the residual oil was

dissolved in 20ml of dry tetrahydrofuran, 0.75g of (Z)-2-amino-α-(methoxyimino)-4-thiazoleacetic acid, 1-hydrobenzotriazole ester (2.3 mmol) was added and the mixture was stirred overnight at room temperature. The solvent was evaporated in vacuo, and the residue was taken up in ethyl acetate, shaken with ice cold aqueous $NaHCO_3$ (1N) and subsequently with ice cold water. The ethyl acetate layer was dried with $Na_2SO_4$ and the title compound was chromatographed on silica gel with ethyl acetate. The oily product (0.68g) was crystallized by treatment with cyclohexane; melting point 74-76°C, dec.

## Example 5

[3S-[3α(Z),4β]]-2-Amino-α-(methoxyimino)-N-[4-methyl-2-oxo-1-(1H-tetrazol-5-ylmethoxy)-3-azetidinyl]-4-thiazoleacetamide

A) (3S-trans)-3-Amino-4-methyl-2-oxo-1-[1H-tetrazol-5-ylmethoxy]azetidine, trifluoroacetate

Deprotection of (3S-trans)-3-[(t-butyloxycarbonyl)amino]-4-methyl-2-oxo-1-[1H-tetrazol-5-ylmethoxy]azetidine according to the procedure of example 4A yielded the title salt.

B) [3S-[3α(Z),4β]]-2-Amino-α-(methoxyimino)-N-[4-methyl-2-oxo-1-(1H-tetrazol-5-ylmethoxy)-3-azetidinyl]-4-thiazoleacetamide

Following the procedure of example 4B, but substituting (3S-trans)-3-amino-4-methyl-2-oxo-1-[1H-tetrazol-5-ylmethoxy]azetidine, trifluoroacetate for (3S-trans)-3-amino-4-methyl-2-oxo-1-[1-(phenylmethyl)-1H-tetrazol-5-ylmethoxy]azetidine,

trifluoroacetate yielded the title compound, melting point 77-81°C, dec.

Example 6

[3S-[3α(Z),4β]]-2-Amino-α-(methoxyimino)-N-[4-methyl-2-oxo-1-(1H-tetrazol-5-ylmethoxy)-3-azetidinyl]-4-thiazoleacetamide, sodium salt

Dropwise addition of aqueous $NaHCO_3$ solution to a suspension of [3S-[3α(Z),4β]]-2-amino-α-(methoxyimino)-N-[4-methyl-2-oxo-1-(1H-tetrazol-5-ylmethoxy)-3-azetidinyl]-4-thiazoleacetamide in water, adjusting the solution to pH 6.5 and freeze-drying yielded the title salt, melting point 218°C.

Example 7

[3S-[3α(Z),4β]]-2-amino-α-(methoxyimino)-N-[4-methyl-2-oxo-1-[1H-tetrazolyl-5-yl(1-ethoxy)]-3-azetidinyl]-4-thiazoleacetamide, sodium salt

A) (3S-trans)-3-Amino-4-methyl-2-oxo-1-[1H-tetrazol-5-yl(1-ethoxy)]azetidine, trifluoroacetate

Deprotection of (3S-trans)-3-[(t-butyloxycarbonyl)amino]-4-methyl-2-oxo-1-[1H-tetrazol-5-yl(1-ethoxy)]azetidine according to the procedure of example 4A yielded the title salt.

B) [3S-[3α(Z),4β]]-2-Amino-α-(methoxyimino)-N-[4-methyl-2-oxo-1-[1H-tetrazol-5-yl(1-ethoxy)-3-azetidinyl]-4-thiazoleacetamide, sodium salt

Following the procedure of example 4B and example 6 but substituting (3S-*trans*)-3-amino-4-methyl-2-oxo-1-[1*H*-tetrazol-5-yl(1-ethoxy)]-azetidine, trifluoroacetate for (3S-*trans*)-3-amino-4-methyl-2-oxo-1-[1-(phenylmethyl)-1*H*-tetrazol-5-ylmethoxy]azetidine, trifluoroacetate yielded the title salt, melting point 226°C, dec.

What we claim is:

1. A compound having the formula

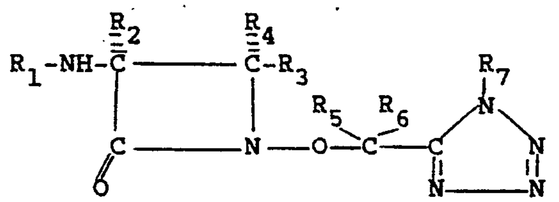

or a pharmaceutically acceptable salt thereof, wherein

$R_1$ is an acyl group derived from a carboxylic acid;

$R_2$ is hydrogen or methoxy;

$R_3$ and $R_4$ are the same or different and each is hydrogen, alkyl, alkenyl, alkynyl, cycloalkyl, phenyl, substituted phenyl or a 4, 5, 6 or 7-membered heterocycle, or one of $R_3$ and $R_4$ is hydrogen and the other is azido, halomethyl, dihalomethyl, trihalomethyl, alkoxycarbonyl, 2-phenylethenyl, 2-phenylethynyl, $-CH_2X_1$, carboxyl, $-S-X_2$, $-O-X_2$, $-A-\overset{O}{\overset{\|}{C}}-NX_6X_7$, $-O-\underset{X_5}{\overset{X_3}{C}}-X_4$ or $-S-\underset{X_5}{\overset{X_3}{C}}-X_4$; wherein $X_1$ is azido, amino, hydroxy, alkanoylamino, phenylcarbonylamino, (substituted phenyl)carbonylamino, alkylsulfonyloxy, phenylsulfonyloxy, (substituted phenyl)sulfonyloxy, phenyl, substituted phenyl, cyano, $-A-\overset{O}{\overset{\|}{C}}-NX_6X_7$, $-S-X_2$ or $-O-X_2$; $X_2$ is alkyl, substituted alkyl, phenyl, substituted phenyl, phenylalkyl, (substituted phenyl)alkyl,

alkanoyl, phenylalkanoyl, (substituted phenyl)-alkanoyl, phenylcarbonyl, (substituted phenyl)-carbonyl, or heteroarylcarbonyl; one of $X_3$ and $X_4$ is hydrogen and the other is hydrogen or alkyl, or $X_3$ and $X_4$ when taken together with the carbon atom to which they are attached form a cycloalkyl group; $X_5$ is formyl, alkanoyl, phenylcarbonyl, (substituted phenyl)carbonyl, phenylalkylcarbonyl, (substituted phenyl)alkylcarbonyl, carboxyl, alkoxycarbonyl, aminocarbonyl, (substituted amino)carbonyl, or cyano; A is $-CH=CH-$, $-(CH_2)_n-$, $-CH_2-O-$, $-CH_2-NH-$ or $-CH_2-S-CH_2$; n is 0, 1 or 2; and $X_6$ and $X_7$ are the same or different and each is hydrogen, alkyl, phenyl or substituted phenyl, or $X_6$ is hydrogen and $X_7$ is amino, substituted amino, acylamino or alkoxy, or $X_6$ and $X_7$ when taken together with the nitrogen atom to which they are attached form a 4, 5, 6 or 7-membered heterocycle;

$R_5$ and $R_6$ are the same or different and each is hydrogen, alkyl, alkenyl, alkynyl, phenyl, substituted phenyl, cycloalkyl or a 4, 5, 6 or 7-membered heterocycle, or $R_5$ and $R_6$ together with the carbon atom to which they are attached are cycloalkyl or a 4, 5, 6 or 7-membered heterocycle, or one of $R_5$ and $R_6$ is hydrogen and the other is azido, halomethyl, dihalomethyl, trihalomethyl, halogen, alkoxycarbonyl, alkenyl, alkynyl, 2-phenylethenyl, 2-phenylethynyl, carboxyl, $-CH_2-X_1$, $-S-X_2$, $-O-X_2$, or $A-\overset{O}{\overset{\|}{C}}-NX_6X_7$; and

$R_7$ is hydrogen, alkyl, phenyl, substituted phenyl, phenylalkyl or (substituted phenyl)alkyl.

2. A compound in accordance with claim 1 wherein $R_2$ is hydrogen.

3. A compound in accordance with claim 2 wherein $R_3$, $R_4$, $R_5$ and $R_6$ are each independently hydrogen or alkyl.

4. A compound in accordance with claim 2 wherein $R_3$, $R_4$, $R_5$ and $R_6$ are each independently hydrogen or methyl.

5. A compound in accordance with claim 2 wherein $R_7$ is hydrogen.

6. A compound in accordance with claim 2 wherein $R_1$ is

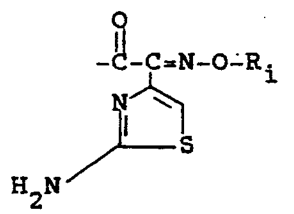

and $R_i$ is methyl, ethyl, carboxymethyl, 1-carboxy-1-methylethyl, 2,2,2-trifluoroethyl or 1-carboxycyclopropyl.

7. A compound in accordance with claim 2 wherein $R_1$ is

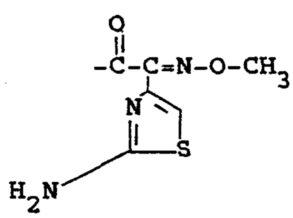

.

8. A compound in accordance with claim 2 wherein $R_1$ is

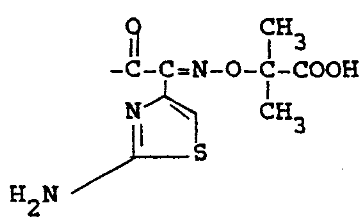

.

9. A compound in accordance with claim 1 (3S-trans)-3-[(t-butyloxycarbonyl)amino]-4-methyl-2-oxo-1-[1-(phenylmethyl)-1H-tetrazol-5-ylmethoxy]-azetidine.

10. A compound in accordance with claim 1 (3S-trans)-3-[(t-butyloxycarbonyl)amino]-4-methyl-2-oxo-1-[1H-tetrazol-5-ylmethoxy]azetidine.

11. A compound in accordance with claim 1 (3S-trans)-3-[(t-butyloxycarbonyl)amino]-4-methyl-2-oxo-1-[1H-tetrazol-5-yl-(1-ethoxy)]-azetidine.

12. A compound in accordance with claim 1 [3S-[3α(Z),4β]]-2-amino-α-(methoxyimino)-N-[4-methyl-2-oxo-1-[[1-(phenylmethyl)-1H-tetrazol-5-yl-methoxy]-3-azetidinyl]-4-thiazoleacetamide.

13. A compound in accordance with claim 1 [3S-[3α(Z),4β]]-2-amino-α-(methoxyimino)-N-[4-methyl-2-oxo-1-(1H-tetrazol-5-ylmethoxy)-3-azetidinyl]-4-thiazoleacetamide.

14. A compound in accordance with claim 1 [3S-[3α(Z),4β]]-2-amino-α-(methoxyimino)-N-[4-methyl-2-oxo-1-[1H-tetrazolyl-5-yl(1-ethoxy)]-3-azetidinyl]-4-thiazoleacetamide.

15. A compound having the formula

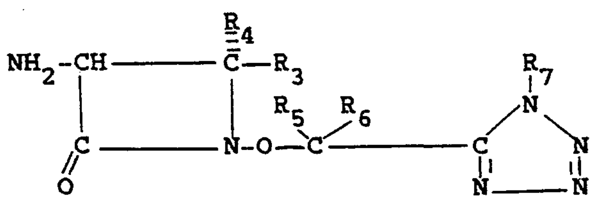

wherein

$R_3$ and $R_4$ are the same or different and each is hydrogen, alkyl, alkenyl, alkynyl, cycloalkyl, phenyl, substituted phenyl or a 4, 5, 6

or 7-membered heterocycle, or one of $R_3$ and $R_4$ is hydrogen and the other is azido, halomethyl, dihalomethyl, trihalomethyl, alkoxycarbonyl, 2-phenylethenyl, 2-phenylethynyl, $-CH_2X_1$,

carboxyl, $-S-X_2$, $-O-X_2$, $-A-\overset{O}{\overset{\|}{C}}-NX_6X_7$,

$-O-\underset{X_5}{\underset{|}{\overset{X_3}{\overset{|}{C}}}}-X_4$ or $-S-\underset{X_5}{\underset{|}{\overset{X_3}{\overset{|}{C}}}}-X_4$; wherein $X_1$ is azido, amino, hydroxy, alkanoylamino, phenylcarbonylamino, (substituted phenyl)carbonylamino, alkylsulfonyloxy, phenylsulfonyloxy, (substituted phenyl)sulfonyloxy, phenyl, substituted phenyl,

cyano, $-A-\overset{O}{\overset{\|}{C}}-NX_6X_7$, $-S-X_2$ or $-O-X_2$; $X_2$ is alkyl, substituted alkyl, phenyl, substituted phenyl, phenylalkyl, (substituted phenyl)alkyl, alkanoyl, phenylalkanoyl, (substituted phenyl)-alkanoyl, phenylcarbonyl, (substituted phenyl)-carbonyl, or heteroarylcarbonyl; one of $X_3$ and $X_4$ is hydrogen and the other is hydrogen or alkyl, or $X_3$ and $X_4$ when taken together with the carbon atom to which they are attached form a cycloalkyl group; $X_5$ is formyl, alkanoyl, phenylcarbonyl, (substituted phenyl)carbonyl, phenylalkylcarbonyl, (substituted phenyl)alkylcarbonyl, carboxyl, alkoxycarbonyl, aminocarbonyl, (substituted amino)carbonyl, or cyano; A is $-CH=CH-$, $-(CH_2)_n-$, $-CH_2-O-$, $-CH_2-NH-$ or $-CH_2-S-CH_2$; n is 0, 1 or 2; and $X_6$ and $X_7$ are the same or different and each is hydrogen, alkyl, phenyl or substituted phenyl, or $X_6$ is hydrogen and $X_7$ is amino, substituted amino,

acylamino or alkoxy, or $X_6$ and $X_7$ when taken together with the nitrogen atom to which they are attached form a 4, 5, 6 or 7-membered heterocycle;

$R_5$ and $R_6$ are the same or different and each is hydrogen, alkyl, alkenyl, alkynyl, phenyl, substituted phenyl, cycloalkyl or a 4, 5, 6 or 7-membered heterocycle, or $R_5$ and $R_6$ together with the carbon atom to which they are attached are cycloalkyl or a 4, 5, 6 or 7-membered heterocycle, or one of $R_5$ and $R_6$ is hydrogen and the other is azido, halomethyl, dihalomethyl, trihalomethyl, halogen, alkoxycarbonyl, alkenyl, alkynyl, 2-phenylethenyl, 2-phenylethynyl, carboxyl, $-CH_2-X_1$, $-S-X_2$, $-O-X_2$, or $A-\overset{O}{\overset{\|}{C}}-NX_6X_7$; and

$R_7$ is hydrogen, alkyl, phenyl, substituted phenyl, phenylalkyl or (substituted phenyl)alkyl.

European Patent Office

# EUROPEAN SEARCH REPORT

0135814
Application number

EP 84 11 0082

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| A | JOURNAL OF THE AMERICAN CHEMICAL SOCIETY, vol. 102, no. 22, 22nd October 1980, pages 7026-7032, American Chemical Society, USA; M.J. MILLER et al.: "Synthesis of beta-lactams from substituted hydroxamic acids" * Page 7028, column 2, paragraph 2 * --- | 1 | C 07 D 403/12<br>C 07 D 417/14 //<br>C 07 D 205/08 |
| P,A | EP-A-0 114 128 (ROUSSEL-UCLAF) * Claims * --- | 1,15 | |
| P,A | FR-A-2 531 427 (SQUIBB) * Claims * ----- | 1,15 | |
| | | | TECHNICAL FIELDS SEARCHED (Int. Cl.4) |
| | | | C 07 D 403/00<br>C 07 D 417/00<br>C 07 D 205/00 |

The present search report has been drawn up for all claims

| Place of search | Date of completion of the search | Examiner |
|---|---|---|
| THE HAGUE | 09-11-1984 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03 82